(19)

Europäisches
Patentamt
European
Patent Office
Office européen
des brevets

(11) **EP 2 335 685 A1**

(12) **EUROPEAN PATENT APPLICATION**
published in accordance with Art. 153(4) EPC

(43) Date of publication:
**22.06.2011 Bulletin 2011/25**

(51) Int Cl.:
*A61K 8/97* (2006.01)      *A61Q 19/02* (2006.01)
*A61Q 19/08* (2006.01)

(21) Application number: **09813119.6**

(22) Date of filing: **10.09.2009**

(86) International application number:
**PCT/JP2009/065848**

(87) International publication number:
**WO 2010/029973 (18.03.2010 Gazette 2010/11)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **12.09.2008 JP 2008235415**

(71) Applicant: **Maruzen Pharmaceuticals Co., Ltd.
Onomichi-shi
Hiroshima 722-0062 (JP)**

(72) Inventors:
• **TOHI, Keiko
   Fukuyama-shi
   Hiroshima 729-3102 (JP)**
• **IWAHASHI, Hiroyasu
   Fukuyama-shi
   Hiroshima 729-3102 (JP)**

(74) Representative: **Hill, Christopher Michael
Page White & Farrer
Bedford House
John Street
London, WC1N 2BF (GB)**

(54) **SKIN-WHITENING AGENT, ANTI-AGING AGENT, AND SKIN COSMETIC**

(57)    A skin-whitening agent or anti-aging agent is provided that contains at least one of bayberry extract, blue flag extract, Japanese pepper extract, cowslip extract, lemon verbena extract, hairy agrimony extract, blueberry extract, celery extract, salt cedar extract and passion fruit extract

EP 2 335 685 A1

**Description**

Technical Field

The present invention relates to a skin-whitening agent and an anti-aging agent, as well as to a skin-care cosmetic agent that contains at least one of the skin-whitening agent and the anti-aging agent.

Background Art

The epidermis and dermis of the skin are composed of epidermal cells, skin fibroblasts and extracellular matrix components such as collagen and elastin that are present outside these cells and serve to support the skin structure. In young skin, interactions between these skin tissue constituents help to maintain homeostasis of the skin and thus help to ensure moisture retention, flexibility, elasticity and other properties of the skin. As a result, a young skin can retain firmness and complexion in its appearance and remain fresh.

However, as the skin is exposed to UV radiation (i.e., UVA and UVB), extremely dry air, excessive skin-cleansing process, hydrogen peroxide and other external factors, and as the skin undergoes the aging process, the production of collagen, elastin and other extracellular matrix components will decrease and the crosslinking of these materials will take place, making the skin less elastic. As a result, the skin loses its ability to retain moisture and its elasticity and the cuticle begins to peel off abnormally. This leads to loss of the skin's firmness and complexion, making the skin susceptible to roughness, wrinkles and other age-related symptoms.

As mentioned above, it is known that the age-related changes in the skin properties, such as wrinkling, dullness, loss of texture and decreased elasticity, involve depletion or denaturation of collagen, elastin and other dermal matrix components.

Of different types of collagens, type-I collagen is the most abundant throughout the body. It is abundantly present in the dermis and is known to play an important role in providing the skin strength.

Elastase, an enzyme that hydrolyzes elastin present in the dermis of the skin, can be over-expressed as a result of exposure to UV radiation or aging. It is believed that the over-expressed enzyme denatures or destroys elastin, causing the loss of skin elasticity.

A class of proteases known as matrix metalloproteinases (which may be referred to as MMPs, hereinafter) has recently attracted much attention as factors that induce depletion or denaturation of the dermal matrix components.

MMPs are classified into the following five groups according to their primary structures and their specificity to different substrates: (1) collagenase group (MMP-1, MMP-8 and MMP-13), (2) gelatinase group (MMP-2 and MMP-9), (3) stromelysin group (MMP-3 and MMP-10), (4) membrane-bound MMPs (MMP-14, MMP-15, MMP-16 and MMP-17), and (5) others (MMP-7, MMP-11 and MMP-12) (See, Patent Literature 1).

Of different MMPs, MMP-1 and MMP-14 are enzymes known to degrade type-I collagen, type-II collagen and type-III collagen, each a major constituent of the dermal matrix of the skin. The expression of these enzymes is significantly enhanced by exposure to UV radiation and is believed to be one of the major causes of UV-related collagen depletion or denaturation that can result in wrinkling and other undesired conditions of the skin.

The stratus corneum is composed of corneocytes, cells formed as a result of terminal differentiation of epidermal keratinocytes, and intercellular lipids that fill the intercellular spaces. The intercellular lipids, which are composed mainly of ceramides, form a lamellar structure that plays an essential role in maintaining the barrier function of stratus corneum. A corneocyte is composed primarily of keratin fibers and is surrounded by a hydrophobic, strong cell-membrane-like structure called a cornified envelope (which is referred to simply as "CE," hereinafter). Serving as a membrane-lining protein, CEs are formed as the CE precursor proteins such as involucrin and loricrin produced in epidermal keratinocytes during their differentiation are cross-linked by the action of an enzyme transglutaminase-1 and become insoluble. CEs are essential for the barrier function of the skin. Further, some ceramides are covalently bound to part of CEs to form a hydrophobic structure that serves as the foundation for the lamellar structure of intercellular lipids. This covalent binding of ceramides forms the basis for the barrier function of the stratus corneum and the ability of the skin to retain moisture.

However, when the turnover time is affected by aging, dryness, UV radiation (UVA, UVB) and other factors, a condition known as incomplete keratinization is induced in which lamellar structures are disturbed and the formation of CEs becomes incomplete. This in turn affects the structure of corneocytes and intercellular lipids and results in decreased water retention and weakened barrier function of the stratus corneum. As a result, skin roughness, dry skin and other age-related skin symptoms will arise. Immature CEs are also frequently observed in patients suffering from psoriasis or atopic dermatitis in their affected skin areas where the barrier function has been reduced. Thus, it is believed that the complete formation of CEs is essential for the barrier function of the skin (See, Non-Patent Literature 1).

Accordingly, if an anti-aging agent can be provided that has the ability to enhance the growth of skin fibroblasts, enhance the production of transglutaminase-1, inhibit elastase activity, inhibit MMP-1 activity, has estrogen-like activity, enhance the production of type-I collagen, enhance the growth of epidermal keratinocytes, and enhance the recovery from UVB damage, as well as other desirable abilities, such an agent would be able to effectively prevent or ameliorate age-related skin symptoms, such as decreased skin elasticity, skin roughness and wrinkling.

While melanin present in the skin serves to protect the body from UV radiation, excessive production or non-uniform deposition of the pigment can cause darkening or blemishes in the skin. The biosynthesis of melanin generally involves the following reactions: tyrosinase, an enzyme synthesized in the melanocytes, first converts tyrosine to DOPA, which in turn is converted to dopaquinone, which is then converted to melanin via intermediates such as 5,6-dihydroxyindophenol. Thus, it is believed that in order to prevent or ameliorate dark skin (i.e., melanopathy), or in order to achieve skin-lightening effect, inhibiting the melanin production process or bleaching the already produced melanin will provide an effective measure.

Several herbal medicines have been reported to have the ability to inhibit tyrosinase activity, including *tocha* extract (See, Patent Literature 2) and water-pepper extract (See, Patent Literature 3).

Until recently, development of skin-whitening agents has focused on tyrosinase, the rate-limiting enzyme in the melanin synthesis. However, it has recently been reported that certain cytokines are produced at increased levels by the epidermal keratinocytes after exposure to UVB radiation and activate melanocytes. Thus, attention has now been shifted to developing agents that provide skin-lightening effect by suppressing melanin production by blocking the signal transduction pathways involving these cytokines. Among such cytokines are $\alpha$-melanocyte-stimulating hormone ($\alpha$-MSH), endothelin-1 (ET-1), nitrogen monoxide (NO), basic fibroblast growth factor (bFGF), granulocyte-macrophage-colony-stimulating factor (GM-SCF) and stem cell factor (SCF). It has been reported that some herbal medicine extracts, such as chamomile extract and Althaea extract, inhibit the effects of endothelin-1 (ET-1) on melanocytes (See, Non-Patent Literature 2).

To date, no skin-whitening agents or anti-aging agents have been provided that are not only made from readily available, inexpensive and safe natural materials, but do not also affect taste, scent, usability or other qualities of the products to which they are added, and that can be used in a wide range of skin-care cosmetic agents. Thus, there is an urgent need for such agents.

Citation List

Patent Literature

PTL1    Japanese Patent Application Laid-Open (JP-A) No. 2000-344672
PTL2    Japanese Patent Application Laid-Open (JP-A) No. 2002-370962
PTL3    Japanese Patent Application Laid-Open (JP-A) No. 2004-083488

Non Patent Literature

NPTL1    Experimental Dermatology 12: 591-601 (2003)
NPTL2    Fragrance Journal, Vol.28, No.9, p65-71 (2000)

Summary of the Invention

Technical Problem

Accordingly, it is a first objective of the present invention to provide a natural skin-whitening agent that has at least one of high tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA, and that is highly safe and can be produced from readily available materials.

It is a second objective of the present invention to provide an anti-aging agent that has at least one of high elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3), and that is highly safe and can be produced from readily available materials.

It is a third objective of the present invention to provide a skin-care cosmetic agent that contains as an active ingredient at least one of the above-described skin-whitening agents and anti-aging agents of the present invention.

Solution to Problem

In an effort to solve the above-described problems, the present inventors have conducted extensive studies to develop a skin-whitening agent and an anti-aging agent that are readily available and inexpensive, are derived from safe natural products, do not affect taste, scent, usability and other qualities of the products to which they are added, and can be used in a wide range of skin-care cosmetic agents. These studies ultimately led to the findings that at least one of bayberry extract, blue flag extract, Japanese pepper extract, cowslip extract, lemon verbena extract, hairy agrimony extract, blueberry extract, celery extract, salt cedar extract and passion fruit extract has (1) at least one of high tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA, each of which activities makes the at least one of the extracts useful as a skin-whitening agent, and has (2) high elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3), each of which activities makes the at least one of the extracts useful as an anti-aging agent.

The present invention is based upon the findings of the present inventors, and means for solving the aforementioned problems are as follows:

<1> A skin-whitening agent, containing:

at least one from the group consisting of bayberry extract, blue flag extract, Japanese pepper extract, cowslip extract, lemon verbena extract, hairy agrimony extract, blueberry extract, celery extract, salt cedar extract and passion fruit extract.

<2> The skin-whitening agent according to <1>, wherein the skin-whitening agent has at least one activity selected from the group consisting of tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA.

<3> An anti-aging agent, containing:

at least one selected from the group consisting of bayberry extract, blue flag extract, Japanese pepper extract, cowslip extract, lemon verbena extract, hairy agrimony extract, blueberry extract, celery extract, salt cedar extract and passion fruit extract.

<4> The anti-aging agent according to <3>, wherein the anti-aging agent has at least one activity selected from the group consisting of elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3).

<5> A skin-care cosmetic agent, containing:

at least one of the skin-whitening agent as defined in any of <1> or <2>, and the anti-aging agent as defined in any of <3> or <4>.

Advantageous Effects of Invention

According to a first aspect of the present invention, there is provided a natural skin-whitening agent that has at least one of high tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA, and that is highly safe and can be produced from readily available materials.

According to a second aspect of the present invention, there is provided an anti-aging agent that has at least one of high elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3), and that is highly safe and can be produced from readily available materials.

According to a third aspect of the present invention, there is provided a skin-care cosmetic agent that contains as an active ingredient at least one of the above-described skin-whitening agents and anti-aging agents of the present invention.

Description of Embodiments

(Skin-whitening agents and Anti-aging Agents)

The skin-whitening agent and the anti-aging agent of the present invention contains at least one of bayberry extract, blue flag extract, Japanese pepper extract, cowslip extract, lemon verbena extract, hairy agrimony extract, blueberry extract, celery extract, salt cedar extract and passion fruit extract, and optional other components.

The skin-whitening agent has superb skin-lightening effects based on at least one of tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA.

While details of skin-lightening substances present in bayberry extract, blue flag extract, Japanese pepper extract, cowslip extract, lemon verbena extract, hairy agrimony extract, blueberry extract, celery extract, salt cedar extract and passion fruit extract are still unknown, the favorable effects of the plant extracts that make them useful as a skin-whitening agent have never been described previously: it is a completely new finding made by the present inventors.

The anti-aging agent of the present invention has superb anti-aging effects based on at least one of elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3).

While details of anti-aging substances present in bayberry extract, blue flag extract, Japanese pepper extract, cowslip extract, lemon verbena extract, hairy agrimony extract, blueberry extract, celery extract, salt cedar extract and passion fruit extract are still unknown, the favorable effects of the plant extracts that make them useful as an anti-aging agent have never been described previously: it is a completely new finding made by the present inventors.

Bayberry *(Myrica cerifera,* Japanese name: Shiro-yamamomo) is a plant belonging to the genus Myrica of the family Myricaceae.

Any part of a bayberry plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, root barks are particularly preferred. These parts are suitably selected depending on the intended purpose.

Japanese pepper *(Piper kadsura,* Japanese name: Futo-kadsura) is a plant belonging to the genus Piper of the family Piperaceae. It is distributed in Japan from the Honsyu island (southern Kanto area and to the west) to Okinawa and can be easily obtained in these areas.

Any part of a Japanese pepper plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, stems are particularly preferred. These parts are suitably selected depending on the intended purpose.

Hairy agrimony *(Agrimonia pilosa,* Japanese name: Kin-mizuhiki) is a perennial plant belonging to the genus Aglimonia of the family Rosaceae. In Japan, it inhabits edges of woods, fields and roadsides of Honsyu, Shikoku and Kyusyu areas and can be easily obtained in these areas.

Any part of a hairy agrimony plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, whole plants are particularly preferred. These parts are suitably selected depending on the intended purpose.

Blueberry *(Vaccinium spp.)* is a collective name for deciduous shrubby berry trees of the genus Vaccinium of the family Ericaceae indigenous to North America.

Any part of a blue berry plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, leaves are particularly preferred. These parts are suitably selected depending on the intended purpose.

Blue flag *(Iris versicolor)* is a plant of the family Iridaceae.

Any part of a blue flag plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, roots are particularly preferred. These parts are suitably selected depending on the intended purpose.

Cowslip *(Primula vernis,* Japanese name: Seiyo sakurasou) is a plant of the family Primulaceae.

Any part of a cowslip plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, leaves are particularly preferred. These parts are suitably selected depending on the intended purpose.

Lemon Verbena *(Aloysia triphylla)* is a deciduous shrub belonging to the genus Aloysia of the family Verbenacae. It grows to a height of 1 m to 3 m and its leaves emit a strong lemon scent.

Any part of a lemon verbena plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, leaves are particularly preferred. These parts are suitably selected depending on the intended purpose.

Celery *(Apium graveolens)* is a plant belonging to the family Apiaceae. The plant was traditionally called in Japan as "Oranda mitsuba" (meaning "Dutch honewort") and "Kiyomasa ninjin." Almost entire part of the plant, including leaves, stalks, roots and seeds, are edible. The plant has a characteristic strong scent.

Any part of a celery plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, seeds are particularly preferred. These parts are suitably selected depending on the intended purpose.

Salt cedar *(Tamarix tenuissima,* Japanese name: Gyoryu) is a plant of the family Tamaricacae.

Any part of a salt cedar plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, flowers are particularly preferred. These parts are suitably selected depending on the intended purpose.

Passion fruit *(Passiflora edulis)* is a plant of the family Passifloraceae.

Any part of a passion fruit plant may be used as the material for extraction, including, but not limited to, flowers, buds, fruits, fruit skins, seeds, seed coats, stems, leaves, branches, leafstalks, trunks, barks, roots, rhizomes, root barks or mixtures thereof. Of these, leaves are particularly preferred. These parts are suitably selected depending on the intended purpose.

Each of the above-described plants used as the extraction material may be dried and directly used in a solvent extraction process, or it may be dried and crushed using, for example, a pulverizer, prior to the solvent extraction process. It is particularly preferred to dry and crush the plant immediately after their collection. The drying process may be carried out by placing the plant under the sun, or by using a commonly used drier. Each of the above-described plants may be pretreated, for example, by degreasing it with a non-polar solvent such as hexane and benzene, prior to extraction. The degreasing and other pretreatments can help improve efficiency of the extraction of the plant using polar solvents.

The extract of each of the above-described plants can be easily obtained by using techniques commonly used in the extraction of plants. Alternatively, the extract of each plant may be a commercially available product. Examples of the extracts of the above-described plants include extracts of the plants, diluted or concentrated extracts, dried extracts or crudely purified or purified products thereof.

The solvent for use in the extraction process may be any solvent suitably selected depending on the intended purpose. For example, water, a hydrophilic organic solvent, or a mixed solvent thereof may preferably be used at room temperature, or at the boiling temperature of the solvent or lower. Skin-lightening or anti-aging components present in each of the above-described plants can be easily extracted from the plants by an extraction process using a polar solvent as an extraction solvent.

Water for use as the extraction solvent may be any type of water suitably selected depending on the intended purpose, including, but not limited to, pure water, tap water, well water, mineral spring water, mineral water, hot spring water, spring water, fresh water and processed water products thereof. Examples of the processes to process the above-described water include purification, heating, sterilization, filtration, ion-exchange, adjustment of osmotic pressure and buffering. Thus, water for use as the extraction solvent includes purified water, hot water, ion-exchanged water, physiological saline, phosphate-buffered solution and phosphate-buffered saline.

The hydrophilic organic solvent for use as the extraction solvent may be any hydrophilic organic solvent suitably selected depending on the intended purpose, including, but not limited to, lower alcohols having 1 carbon atom to 5 carbon atoms, such as methanol, ethanol, propyl alcohol and isopropyl alcohol; lower aliphatic ketones, such as acetone and methyl ethyl ketone; and polyhydric alcohols having 2 carbon atoms to 5 carbon atoms, such as 1,3-butylene glycol, propylene glycol and glycerol. A mixed solvent of any of these hydrophobic organic solvents and the above-described water may also be used. When used, the mixed solvent of water and the hydrophobic organic solvent may preferably contain 1 part by mass to 90 parts by mass of any of the above-described lower alcohols with respect to 10 parts by mass of water, or it may preferably contain 1 part by mass to 40 parts by mass of any of the above-described lower ketones with respect to 10 parts by mass of water. The mixed solvent may also preferably contain 1 part by mass to 90 parts by mass of any of the above-described polyols with respect to 10 parts by mass of water.

Extraction of the extracts from the above-described plants used as the extraction material does not require special extraction techniques and may be carried out by using any suitable extraction apparatus at room temperature or at refluxing temperature.

Specifically, the desired extract may be obtained in the following manner. The extraction material is first added to a processing tank containing the extraction solvent. The mixture is then allowed to stand for 30 minutes to 4 hours while occasionally stirred as necessary. This allows the soluble components to dissolve into the solvent. Subsequently, the mixture is filtered to remove the solid and the resulting extract is distilled to remove the extraction solvent. The resulting residue is then dried to give the desired extract. The amount of the extraction solvent is typically from 5 times to 15 times the amount of the extraction material (by mass). When the extraction solvent is water, extraction is typically carried out at 50°C to 95°C for about 1 hour to about 4 hours. When the extraction solvent is a mixed solvent of water and ethanol, extraction is typically carried out at 40°C to 80°C for about 30 minutes to about 4 hours. The extract obtained through the extraction with the solvent may be directly used as an active ingredient of the skin-whitening agent or the skin-aging agent of the present invention, given that the extraction solvent used is highly safe.

In order to obtain the diluted or concentrated extracts, dried extracts or crudely purified or purified products thereof, the extracts of the above-described plants resulting from the extraction process may be subjected to dilution, concentration, drying, purification or other suitable processes using commonly used techniques. While the extracts of the above-described plants may be directly used as an active ingredient of the skin-whitening agent or the anti-aging agent, the concentrated extracts or dried extracts are more convenient. The dried extracts can be obtained by using commonly used techniques. Carriers such as dextrin and cyclodextrin may be added to improve the absorption of moisture. Since the above-described plants used as the extraction material may have characteristic scents or tastes, the plant extracts may be further purified for discoloration or deodorization to the extent that the physiological activity of the plants is not reduced. In practice, however, unpurified extracts may be used in, for example, skin-care cosmetic agents without causing any problems since the amount used in these products is relatively small. Specifically, the purification process may be carried out by using active carbon, adsorption resins, ion-exchange resins or other suitable materials.

The plant extracts obtained in the above-described manner have at least one of the following activities: tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA, elastase inhibitory activity, MMP-1 inhibitory activity, activity to enhance ATP production, activity to enhance the production of laminin-5, activity to enhance the production of filaggrin, activity to enhance the production of transglutaminase-1, activity to enhance the expression of mRNA of hyaluronic acid synthase-3 (HAS3), activity to enhance the expression of mRNA of aquaporin-3 (AQP3), activity to enhance the production of type-I collagen, activity to enhance the production of type-IV collagen, activity to enhance the growth of skin fibroblasts, activity to enhance recovery from damage caused by UVB radiation, activity to enhance the growth of epidermal keratinocytes, activity to enhance the production of involucrin, and activity to suppress damage caused by hydrogen peroxide. These activities make each of the plant extracts suitable for use in the skin-whitening agent or the anti-aging agent of the present invention.

The extracts of the above-described plants may be used in the skin-whitening agent or the anti-aging agent in any amount suitably selected depending on the intended purpose. The extracts of the above-described plants may be used as the skin-whitening agent or the anti-aging agent by themselves.

The skin-whitening agent or the anti-aging agent may contain any one or two or more of the extracts of the above-described plants. When two or more of the plant extracts are present in the skin-whitening agent or the anti-aging agent, they may be contained at any suitable ratio selected depending on the intended purpose.

As long as the advantages of the present invention are not affected, the skin-whitening agent or the anti-aging agent may contain optional components other than the above-described plant extracts. Such optional components can include any component suitably selected depending on the intended purpose, one example being physiological saline for diluting the plant extracts to a desired concentration. The skin-whitening agent or the anti-aging agent may contain each of these optional components at any amount suitably selected depending on the intended purpose.

If necessary, the skin-whitening agent or the anti-aging agent may be formulated in the form of powders, granules, tablets or other suitable dosage forms.

Not only does the skin-whitening agent or the anti-aging agent of the present invention have superb skin-lightning effects and anti-aging effects, but they also offer high usability and safety that make them particularly suitable for use in the skin-care cosmetic agent of the present invention.

(Skin-care cosmetic agents)

The skin-care cosmetic agent of the present invention contains as an active ingredient at least one of the skin-whitening agent or the anti-aging agent of the present invention, along with other suitably selected optional components.

The skin-care cosmetic agent may be used in various suitably selected applications, including, but not limited to, ointments, creams, emulsions, lotions, packs, jelly, lip creams, lipsticks, bath agents, astringents and other suitable applications.

While the amount of the skin-whitening agent or the anti-aging agent added to the skin-care cosmetic agent may be suitably adjusted depending on the type of the skin-care cosmetic agent, the physiological activity of the extracts and other factors, the amount is preferably from 0.0001% by mass to 10% by mass, and more preferably from 0.001% by mass to 1% by mass as measured in the amount of the plant extracts.

As long as the intended purposes and the advantages of the present invention are not affected, the skin-care cosmetic agent may contain various optional main agents, auxiliary agents and other components commonly used in the production of skin-care cosmetic agents.

The optional other components include any component that does not interfere with the skin-lightening effects or the anti-aging effects of the present invention and may be selected depending on the intended purpose. Examples of other components include, but are not limited to, astringents, anti-bacterial agents, antifungal agents, UV-absorbing agents, humectants, cell-stimulating agents, oils and fats, waxes, hydrocarbons, fatty acids, alcohols, esters, surfactants, flavors and other suitable components.

The skin-care cosmetic agent of the present invention is highly safe when applied to the skin and exhibits at least one of high tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA, elastase inhibitory activity, MMP-1 inhibitory activity, activity to enhance ATP production, activity to enhance the production of laminin-5, activity to enhance the production of filaggrin, activity to enhance the production of transglutaminase-1, activity to enhance the expression of mRNA of hyaluronic acid synthase-3 (HAS3), activity to enhance the expression of mRNA of aquaporin-3 (AQP3), activity to enhance the production of type-I collagen, activity to enhance the production of type-IV collagen, activity to enhance the growth of skin fibroblasts, activity to enhance recovery from damage caused by UVB radiation, activity to enhance the growth of epidermal keratinocytes, activity to enhance the production of involucrin, and activity to suppress damage caused by hydrogen peroxide.

While the skin-whitening agents, anti-aging agents and skin-care cosmetic agents of the present invention are suitable for use with humans, they may be used with animals other than humans as long as their advantageous effects are obtained.

Examples

The present invention will now be described with reference to examples, which are not intended to limit the scope of the invention in any way.

(Production Example 1)

Production of water extracts of plants

To the crushed product of each of the plants listed in Table 1 below, 10 times as much mass of water was added and the mixture was heated at 80°C for 2 hours and then filtered. To the resulting residue, the same amount of water to that of the residue was added and the mixture was heated at 80°C for 2 hours and then filtered. The resulting filtrate was concentrated and freeze-dried to obtain a desired water extract of each plant (i.e., freeze-dried product).
The extraction rate (%) of each of the water extracts of the respective plants is shown in Table 1.

Table 1

| Extract No. | Plants | Parts | % Extraction |
|---|---|---|---|
| 1 | Bayberry ;*Myrica cerifera* | root bark | 29.8 |
| 2 | B lue flag ;*Iris vesicolor* | root | 25.4 |
| 3 | Japanese Pepper ;*Piperkadzura* | stem | 7.2 |
| 4 | Cowslip ;*Primula veris* | leaf | 25.6 |
| 5 | Lem on Verbena ;*Abysia triphylla* | leaf | 24.7 |
| 6 | H airy A grim ony ;*Agrimonia pilosa* | whole plant | 20.4 |
| 7 | Blueberry ;*Vaccinium spp.* | leaf | 26.8 |
| 8 | Celery ;*Apium graveolens* | seed | 19.8 |
| 9 | Salt Ceder ;*Tamarix tenuissima* | fbw er | 12.5 |
| 10 | Passion Fruit ;*Passiflora edulis* | leaf | 28.8 |

(Production Example 2)

Production of 50% by mass ethanol extract of plants

To the crushed product of each of the plants listed in Table 2 below, 10 times as much mass of 50% by mass ethanol was added and the mixture was heated at 80°C for 2 hours and then filtered. To the resulting residue, the same amount of 50% by mass ethanol to that of the residue was added and the mixture was heated at 80°C for 2 hours and then filtered. The resulting filtrate was concentrated and freeze-dried to obtain a desired 50% by mass ethanol extract of each plant (i.e., freeze-dried product).
The extraction rate (%) of each of the 50% by mass ethanol extracts of the respective plants is shown in Table 2.

Table 2

| Extract No. | Plants | Parts | % Extraction |
|---|---|---|---|
| 1 | Bayberry ;*Myrica cerifera* | root bark | 26.2 |
| 2 | Blue flag ;*Iris vesicolor* | root | 19.6 |
| 3 | Japanese Pepper ;*Piper kadzura* | stem | 4.6 |
| 4 | Cowslip ;*Primula veris* | leaf | 21.2 |
| 5 | Lem on Verbena ;*Abysia triphylla* | leaf | 19.1 |
| 6 | H airy A grin ony ;*Agrimonia pilosa* | whole plant | 13.8 |
| 7 | Blueberry ;*Vaccinium spp.* | leaf | 23.5 |
| 8 | Celery ;*Apium graveolens* | seed | 14.7 |
| 9 | S alt Ceder ;*Tamarix tenuissima* | fbw er | 9.3 |
| 10 | Passion Fruit ;*Passiflora edulis* | leaf | 23.7 |

(Production Example 3)

Production of 80% by mass ethanol extract of plants

To the crushed product of each of the plants listed in Table 3 below, 10 times as much mass of 80% by mass ethanol was added and the mixture was heated at 80°C for 2 hours and then filtered. To the resulting residue, the same amount of 80% by mass ethanol to that of the residue was added and the mixture was heated at 80°C for 2 hours and then filtered. The resulting filtrate was concentrated and freeze-dried to obtain a desired 80% by mass ethanol extract of each plant (i.e., freeze-dried product).

The extraction rate (%) of each of the 80% by mass ethanol extracts of the respective plants is shown in Table 3.

Table 3

| Extract No. | Plants | Parts | % Extraction |
|---|---|---|---|
| 1 | Bayberry ;*Myrica cerifera* | root bark | 20.5 |
| 2 | Blue flag ;*Iris vesicolor* | root | 12.6 |
| 3 | Japanese Pepper ;*Piper kadzura* | stem | 3.0 |
| 4 | Cowslip ;*Primula veris* | leaf | 18.6 |
| 5 | *Lemon Verbena ,Aloysia triphylla* | leaf | 12.4 |
| 6 | Hairy Agrim ony ;*Agrimonia pilosa* | whole plant | 10.3 |
| 7 | Blueberry ;*Vaccinium spp.* | leaf | 19.6 |
| 8 | Celery ;*Apium graveolens* | seed | 10.5 |
| 9 | S alt Ceder ;*Tamarix tenuissim a* | flower | 6.0 |
| 10 | Passion Fruit ;*Passiflora edulis* | leaf | 19.6 |

(Example 1)

Test for the tyrosinase inhibitory activity

The extracts shown in Table 4 below were used as samples and each sample was tested for the tyrosinase inhibitory activity in the following manner.

McIlvaine buffer (pH6.8) (0.2 mL), 0.06 mL of a 0.3mg/mL tyrosine solution, and 0.18 mL of a 25% by mass DMSO solution of one of the samples were placed in each well of a 48-well plate and the plate was left at 37°C for 10 minutes. A 800 units/mL tyrosinase solution (0.02 mL) was then added and the plate was incubated at 37°C for 15 minutes. After the incubation period, the absorbance was measured at 475 nm. A blank test was conducted in the same manner for correction.

The tyrosinase inhibitory activity was determined by the following equation:

$$\% \text{ tyrosinase inhibition} = \{1 \cdot (St \cdot Sb)/(Ct \cdot Cb)\} \times 100$$

In the equation above, St represents the absorbance of the sample solution at 475 nm; Sb represents the absorbance of the sample blank solution at 475 nm; Ct represents the absorbance of the control solution at 475 nm; and Cb represents the absorbance of the control blank solution at 475 nm.

The results shown in Table 4 below are given by % inhibition at sample concentrations of 100 $\mu$g/ml and 400 $\mu$g/ml, respectively, or by the sample concentration that resulted in 50% inhibition.

Table 4

| | 50% inhibition sample conc.($\mu$g/mL) |
|---|---|
| 50% by m ass ethanolextract of Japanese pepper | 197.3 |
| 50% by m ass ethanolextract of salt cedar | 429.0 |

(continued)

| | 50% inhibition sample conc.(μg/mL) |
|---|---|
| | |
| | % inhibition at 100 μg/mL sample |
| 50% by mass ethanolextract of bayberry | 35.8 |
| | |
| | % inhibition at 400 μg/mL sample |
| 50% by m ass ethanolextract of cow slip | 26.0 |

The results of Table 4 indicate that the bayberry extract, the Japanese pepper extract, the cowslip extract and the salt cedar extract each have an activity to inhibit tyrosinase.

(Example 2)

Test for the activity to suppress melanin production

The extracts shown in Table 5 below were used as samples and each sample was tested for the activity to suppress melanin production in the following manner.

B16 melanoma cells were cultured in Dulbecco's MEM medium supplemented with 10% by mass FBS. The cells were then harvested by trypsin treatment. The harvested cells were diluted with Dulbecco's MEM medium supplemented with 10% by mass FBS and 1 mmol/L theophylline to a concentration of $24.0 \times 10^4$ cells/ml. 300 μL of diluted cells were plated in each well of a 48-well plate and cells were incubated for 6 hours. After the incubation period, 300μL of each sample dissolved in Dulbecco's MEM medium supplemented with 10% by mass FBS and 1mmol/L theophylline was added to each well and cells were incubated for 4 days. After the incubation period, the medium was removed from each well and 200μm of a 1mol/L NaOH solution was added. Cells were then lysed by a sonicator and the absorbance was measured at 475nm. The absorbance at 540nm was also measured as the turbidity. The difference between the two values was then taken to give a measurement of the melanin production.

As a control, cells incubated only with Dulbecco's MEM supplemented with 1mmol/L theophylline were tested in the same manner.

The tyrosinase inhibitory activity was determined by the following equation:

$$\% \text{ suppression of melanin production} = \{1 - (B/D)/(A/C)\} \times 100$$

wherein A represents the absorbance at 475nm in the absence of sample; B represents at 475nm in the presence of sample; C represents the absorbance at 540nm in the absence of sample; and D represents at 540nm in the presence of sample.

The results shown in Table 5 below are given by % inhibition at sample concentrations of 25μg/ml, 100μg/ml, 200μg/ml and 400μg/ml, respectively, or by the sample concentration that resulted in 50% inhibition.

Table 5

| | 50% inhibition sample conc. (μg/mL) |
|---|---|
| 50% by mass ethanolextract of blue flag | 201.0 |
| | |
| | % suppression at 25 μg/mL sample |
| 50% by m ass ethanolextract of hairy agrimony | 33.8 |
| | |

(continued)

|  | % suppression at 100 $\mu$g/mL sample |
| --- | --- |
| 50% by m ass ethanolextract of bayberry | 38.5 |
|  |  |
|  | % suppression at 200 $\mu$g/mL sample |
| 50% by mass ethanolextract of celery | 47.5 |
|  |  |
|  | % suppression at 400 $\mu$g/mL sample |
| 50% by mass ethanol extract of cowslip | 34.9 |
| 50% by m ass ethanol extract of blueberry | 12.4 |
| 50% by m ass ethanol extract of passion fuit | 16.6 |

The results of Table 5 indicate that the blue flag extract, the bayberry extract, the cowslip extract, the hairy agrimony extract, the blueberry extract, the passion fruit extract, and the celery extract each have an activity to inhibit tyrosinase.

(Example 3)

Test for the activity to suppress the expression of endothelin-1 (ET-1) mRNA

The extracts shown in Table 6 below were used as samples and each sample was tested for the activity to suppress the increase in the expression of endothelin-1 mRNA in the following manner.
Normal human neonatal epidermal keratinocytes (NHEK) were pre-cultured in an 80 cm$^2$ flask containing EpiLife-KG2 medium (a culture medium designed for long-term culture of NHEK) at 37°C under 5% $CO_2$. Cells were then harvested by trypsin treatment.
Cells were then plated onto EpiLife-KG2 in a 35mm petri dish (FALCON) at $40 \times 10^4$ cells/2mL/dish and were then incubated at 37°C under 5% $CO_2$ overnight. After 24 hours, the medium was discarded and 1 mL of HEPES buffer was added. Cells were then irradiated with UVB radiation (50mJ/cm$^2$). Subsequently, 2mL of each of the samples dissolved in EpiLife-KG2 to a desired concentration (shown in Table 6) was added to each petri dish and cells were further incubated at 37°C under 5% $CO_2$ for 24 hours. After the incubation period, the medium was discarded and total RNA was extracted using ISOGEN (Wako, Cat. No. 311-02501). The amount of RNA for each culture was measured by a spectrophotometer and total RNA was prepared to a concentration of 200ng/$\mu$L.
Using the total RNA as a template, the expression levels of endothelin-1 (ET-1) mRNA and GAPDH mRNA to serve as the internal standard were determined. The detection was carried out by performing real-time RT-PCR using Takara SYBR ExScript RT-PCR Kit (Perfect Real Time) on a real-time PCR system (Smart Cycler®, Cepheid).
The expression levels of ET-1 were determined as follows: total RNA was obtained from the following cell cultures incubated under different conditions: cells incubated without UV irradiation in the absence of the sample, cells incubated with UV irradiation in the absence of the sample, and cells incubated with UV irradiation in the presence of the sample. The expression levels of the respective total RNA were corrected relative to GAPDH expression. The expression levels for cells incubated with UV irradiation in the absence of the sample and cells incubated with UV irradiation in the presence of the sample were then corrected relative to the expression levels for cells incubated without UV irradiation in the absence of the sample (= 100).
The results were used in the following equation 1 to determine the suppression rate of ET-1 mRNA expression for each sample. Table 6 shows the suppression rate of ET-1 mRNA expression for each sample at a sample concentration of 10$\mu$g/mL or 1$\mu$g/mL.

(Equation 1)

$$\text{\% suppression of endothelin-1 (ET-1) mRNA expression} = \{(A\text{-}B)\text{-}(A\text{-}C)\}/(A\text{-}B) \times 100$$

In the equation 1, A represents the corrected expression level for cells incubated without UV irradiation in the absence of the sample; B represents the corrected expression level for cells incubated with UV irradiation in the absence of the sample; and C represents the corrected expression level for cells incubated with UV irradiation in the presence of the sample.

Table 6

| | % suppression of ET-1 mRNA expression | |
|---|---|---|
| | Sample Conc. = 10 $\mu$g/mL | Sample Conc. = 1$\mu$g/mL |
| 50°/ by mass ethanol extract of Japanese pepper | 151.7 | - |
| 50% by mass ethanol extract of cowslip | 76.0 | - |
| 50% by mass ethanol extract of lem on verbena | 58.1 | - |
| 50% by m ass ethanol extract of hairy agrim ony | 53.8 | - |
| 50% by mass ethanol extract of blueberry | - | 43.0 |
| 50% by mass ethanol extract of celery | - | 18.2 |

The results of Table 6 indicate that the Japanese pepper extract, the cowslip extract, the lemon verbena extract, the hairy agrimony extract, the blueberry extract, and the celery extract each have an activity to inhibit the expression of endothelin-1 (ET-1) mRNA.

(Example 4)

Test for the activity to suppress the expression of SCF mRNA

The extracts shown in Table 7 below were used as samples and each sample was tested for the activity to suppress the expression of SCF mRNA in the following manner.
Normal human neonatal epidermal keratinocytes (NHEK) were pre-cultured in an 80cm$^2$ flask containing EpiLife-KG2 medium (a culture medium designed for long-term culture of NHEK) at 37°C under 5% $CO_2$. Cells were then harvested by trypsin treatment.
Cells were then plated onto a 35mm petri dish (FALCON) in EpiLife-KG2 at $40 \times 10^4$ cells/2mL/dish and were then incubated at 37°C under 5% $CO_2$ overnight. After 24 hours, the medium was discarded and 1mL of HEPES buffer was added. Cells were then irradiated with UVB radiation (50mJ/cm$^2$). Subsequently, 2mL of each of the samples dissolved in EpiLife-KG2 to a desired concentration (shown in Table 7) was added to each petri dish and cells were further incubated at 37°C under 5% $CO_2$ for 24 hours. After the incubation period, the medium was discarded and total RNA was extracted using ISOGEN (Wako, Cat. No. 311-02501). The amount of RNA for each culture was measured by a spectrophotometer and total RNA was prepared to a concentration of 200 ng/$\mu$L.
Using the total RNA as a template, the expression levels of SFC mRNA and GAPDH mRNA to serve as the internal standard were determined. The detection was carried out by performing real-time RT-PCR on a real-time PCR system (Smart Cycler®, Cepheid).
The expression levels of SCF were determined as follows: total RNA was obtained from the following cell cultures incubated under different conditions: cells incubated without UV irradiation in the absence of the sample, cells incubated with UV irradiation in the absence of the sample, and cells incubated with UV irradiation in the presence of the sample. The expression levels of the respective total RNA were corrected relative to GAPDH expression. The expression levels for cells incubated with UV irradiation in the absence of the sample and cells incubated with UV irradiation in the presence of the sample were then corrected relative to the expression levels for cells incubated without UV irradiation in the absence of the sample (= 100).
The results were used in the following equation 2 to determine the suppression rate of SCF mRNA expression for each sample. Table 7 shows the suppression rate of SCF mRNA expression for each sample at a sample concentration of 10μg/mL or 1μg/mL.

$$(\text{Equation 2})$$

$$\% \text{ suppression of SCF mRNA expression} = \{(A{\cdot}B){\cdot}(A{\cdot}C)\}/(A{\cdot}B) \times 100$$

In the equation 2, A represents the corrected expression level for cells incubated without UV irradiation in the absence of the sample; B represents the corrected expression level for cells incubated with UV irradiation in the absence of the sample; and C represents the corrected expression level for cells incubated with UV irradiation in the presence of the sample.

Table 7

|  | % suppression of SFC m RNA expression | |
|---|---|---|
|  | Sample Conc. =10$\mu$ g/mL | Sample Conc.= = 1$\mu$ g/mL |
| 50% by m ass ethanol extract of baybeny | 134.2 | - |
| 50% by m ass ethanol extract of Japanese pepper | 84.0 | - |
| 50% by m ass ethanol extract of cowslip | 83.9 | - |
| 50% by mass ethanol extract of lem on verbena | 57.8 | - |
| 50% by mass ethanol extract of hairy agrimony | 62.9 | - |
| 50% by m ass ethanol extract of blueberry | - | 39.9 |
| 50% by mass ethanol extract of celery | - | 58.4 |

The results of Table 7 indicate that the bayberry extract, the Japanese pepper extract, the cowslip extract, the lemon verbena extract, the hairy agrimony extract, the blueberry extract, and the celery extract each have an activity to inhibit the expression of SCF mRNA.

(Example 5)

Test for the activity to enhance the expression of bFGF mRNA

The extracts shown in Table 8 below were used as samples and each sample was tested for the activity to enhance the expression of bFGF mRNA in the following manner.

Normal human neonatal epidermal keratinocytes (NHEK) were pre-cultured in an 80cm$^2$ flask containing EpiLife-KG2 medium (a culture medium designed for long-term culture of NHEK) at 37°C under 5% $CO_2$-95% air. Cells were then harvested by trypsin treatment.

Cells were then plated onto EpiLife-KG2 in a 35mm petri dish (FALCON) at $40 \times 10^4$ cells/2mL/dish and were then incubated at 37°C under 5% $CO_2$-95% air overnight. After 24 hours, the medium was discarded and 1 mL of HEPES buffer was added. Cells were then irradiated with UVB radiation (50mJ/cm$^2$). Subsequently, 2mL of each of the samples dissolved in EpiLife-KG2 to a desired concentration (shown in Table 8) was added to each petri dish and cells were further incubated at 37°C under 5% $CO_2$-95% air for 24 hours. After the incubation period, the medium was discarded and total RNA was extracted using ISOGEN (Nippon Gene, Cat. No. 311-02501). The amount of RNA for each culture was measured by a spectrophotometer and total RNA was prepared to a concentration of 200ng/μL.

Using the total RNA as a template, the expression levels of bFGF mRNA and GAPDH mRNA to serve as the internal standard were determined. The detection was carried out by performing real-time 2-step RT-PCR using Takara SYBR PrimeScript RT-PCR Kit (Perfect Real Time, code No. RR063A, TAKARA-BIO) on a Smart Cycler real-time PCR system (Cepheid).

The expression levels of bFGF mRNA were determined as follows: total RNA was obtained from the following cell cultures incubated under different conditions: cells incubated without UV irradiation in the absence of the sample, cells incubated with UV irradiation in the absence of the sample, and cells incubated with UV irradiation in the presence of the sample. The expression levels of the respective total RNA were corrected relative to GAPDH expression. The expression levels for cells incubated with UV irradiation in the absence of the sample and cells incubated with UV irradiation in the presence of the sample were then corrected relative to the expression levels for cells incubated without UV irradiation in the absence of the sample (= 100).

The results were used in the following equation to determine the suppression rate (%) of the increase in bFGF mRNA expression for each sample. The results are shown in Table 8.

$$\text{\% suppression of increase in bFGF mRNA expression} = \{(A \cdot B) \cdot (A \cdot C)\}/(A \cdot B) \times 100$$

wherein A represents the corrected expression level for cells incubated without UV irradiation in the absence of the sample; B represents the corrected expression level for cells incubated with UV irradiation in the absence of the sample; and C represents the corrected expression level for cells incubated with UV irradiation in the presence of the sample.

Table 8

|  | % suppression at sample conc. of 10 $\mu$g/mL |
| --- | --- |
| 50% by mass ethanol extract of cowslip | 88.3 |
| 50% by m ass ethanol extract of lemon verbena | 11.7 |
| 50% by m ass ethanol extract of hairy agrimony | 98.2 |
| 50% by mass ethanol extract of blueberry | 41.8 |

The results of Table 8 indicate that the cowslip extract, the lemon verbena extract, the hairy agrimony extract and the blueberry extract each have an activity to enhance the expression of bFGF mRNA.

(Example 6)

Test for the activity to enhance the expression of POMC mRNA

The extracts shown in Table 9 below were used as samples and each sample was tested for the activity to enhance the expression of POMC mRNA in the following manner.

Normal human neonatal epidermal keratinocytes (NHEK) were pre-cultured in an 80cm$^2$ flask containing EpiLife-KG2 medium (a culture medium designed for long-term culture of NHEK) at 37°C under 5% $CO_2$-95% air. Cells were then harvested by trypsin treatment.

Cells were then plated onto EpiLife-KG2 in a 35mm petri dish (FALCON) at $40 \times 10^4$ cells/2mL/dish and were then incubated at 37°C under 5% $CO_2$-95% air overnight. After 24 hours, the medium was discarded and 1 mL of HEPES buffer was added. Cells were then irradiated with UVB radiation (50mJ/cm$^2$). Subsequently, 2mL of each of the samples dissolved in EpiLife-KG2 to a desired concentration (shown in Table 9) was added to each petri dish and cells were further incubated at 37°C under 5% $CO_2$-95% air for 24 hours. After the incubation period, the medium was discarded and total RNA was extracted using ISOGEN (Nippon Gene, Cat. No. 311-02501). The amount of RNA for each culture was measured by a spectrophotometer and total RNA was prepared to a concentration of 200ng/$\mu$L.

Using the total RNA as a template, the expression levels of POMC mRNA and GAPDH mRNA to serve as the internal standard were determined. The detection was carried out by performing real-time 2-step RT-PCR using Takara SYBR PrimeScript RT-PCR Kit (Perfect Real Time, code No. RR063A, TAKARA-BIO) on a Smart Cycler real-time PCR system (Cepheid).

The expression levels of POMC mRNA were determined as follows: total RNA was obtained from the following cell cultures incubated under different conditions: cells incubated without UV irradiation in the absence of the sample, cells incubated with UV irradiation in the absence of the sample, and cells incubated with UV irradiation in the presence of the sample. The expression levels of the respective total RNA were corrected relative to GAPDH expression. The expression levels for cells incubated with UV irradiation in the absence of the sample and cells incubated with UV irradiation in the presence of the sample were then corrected relative to the expression levels for cells incubated without UV irradiation in the absence of the sample (= 100).

The results were used in the following equation to determine the suppression rate (%) of the increase in POMC mRNA expression for each sample. The results are shown in Table 9.

$$\text{\% suppression of increase in POMC mRNA expression} = \{(A\text{-}B)\text{-}(A\text{-}C)\}/(A\text{-}B) \times 100$$

In the equation above, A represents the corrected expression level for cells incubated without UV irradiation in the absence of the sample; B represents the corrected expression level for cells incubated with UV irradiation in the absence of the sample; and C represents the corrected expression level for cells incubated with UV irradiation in the presence of the sample.

Table 9

|  | % suppression at sample conc. of 10 $\mu$g/m L |
|---|---|
| 50% by mass ethanol extract of cowslip | 60.1 |
| 50% by mass ethanol extract of lem on verbena | 49.1 |
| 50% by m ass ethanol extract of hairy agrimony | 11.6 |
|  |  |
|  | % suppression at sample conc. of 1 $\mu$g/m L |
| 50% by m ass ethanol extract of blueberry | 82.0 |
| 50% by mass ethanol extract of celery | 68.5 |

The results of Table 9 indicate that the cowslip extract, the lemon verbena extract, the hairy agrimony extract, the blueberry extract and the celery extract each have an activity to enhance the expression of POMC mRNA.

(Example 7)

Test for the elastase inhibitory activity

The extracts shown in Table 10 below were used as samples and each sample was tested for the elastase inhibitory activity in the following manner.

50$\mu$L of a solution of each sample prepared in 0.2mol Tris-HCl buffer (pH8.0) and 50$\mu$L of a 20$\mu$g/mL solution of type-III elastase were mixed together in a well of a 96-well microplate. 100$\mu$L of a 0.4514mg/mL solution of N-succinyl-Ala-Ala-Ala-nitroanilide prepared in the same buffer was then added and the plate was incubated at 25°C for 15 min. After the incubation period, the absorbance was measured at 415nm. A blank test was conducted in the same manner for correction.

The results were used in the following equation 3 to determine the elastase inhibitory activity:

$$\text{(Equation 3)}$$

$$\text{\% elastase inhibition} = [1\text{-}(C\text{-}D)/(A\text{-}B)] \times 100$$

In the equation 3, A represents the absorption at 415nm of a solution incubated in the absence of the sample and in the presence of the enzyme; B represents the absorption at 415nm of a solution incubated in the absence of both the sample and the enzyme; C represents the absorption at 415nm of a solution incubated in the presence of both the sample and the enzyme; and D represents the absorption at 415nm of a solution incubated in the presence of the sample and in the absence of the enzyme.

Next, the concentration of each sample solution was decreased stepwise and the elastase inhibition (%) was measured. $IC_{50}$ ($\mu$g/mL) (the concentration that results in 50% inhibition) was determined by curve fitting (i.e., the smaller the $IC_{50}$ value, the more potent the elastase inhibitory activity of a given sample). The results are shown in Table 10.

Table 10

|  | IC 50 ($\mu$g/m L) |
|---|---|
| 50% by mass ethanol extract of bayberry | 192.7 |
| 50% by mass ethanol extract of Japanese pepper | 307.8 |

The results of Table 10 indicate that the bayberry extract and the Japanese pepper extract each have an activity to inhibit elastase.

(Example 8)

Test for the activity to inhibit matrix metalloproteinase (MMP-1)

The extracts shown in Table 11 below were used as samples and each sample was tested for the matrix metalloproteinase (MMP-1) inhibitory activity in the manner described below. The test method was devised by partially modifying the method of Wunsch and Heidrich.

50$\mu$L of a solution of each sample dissolved in a 0.1mol/L Tris-HCl buffer (pH7.1) containing 20mmol/L calcium, 50$\mu$L of an MMP-1 solution and 400$\mu$L of a Pz-peptide solution were mixed in a capped test tube and the mixture was incubated at 37°C for 30 min. Subsequently, 1mL of a 25mmol/L citric acid solution was added to stop the reaction. 5mL ethyl acetate was then added and the mixture was agitated vigorously. The mixture was centrifuged (1,600$\times$g, 10 min) and the absorbance of the ethyl acetate layer was measured at 320nm. A blank test was conducted in the similar manner for correction.

MMP-1 used was collagenase type IV derived from *Clostridium histolyticum* (Sigma).

Pz-peptide used was Pz-Pro-Leu-Gly-Pro-D-Arg-OH (BACHEM Fenichemikalien AG).

The results were used in the following equation 4 to determine the MMP-1 inhibitory activity:

$$(\text{Equation 4})$$

$$\% \text{ MMP-1 inhibition} = [1\text{-}(C\text{-}D)/(A\text{-}B)] \times 100$$

In the equation 4, A represents the absorption at 320nm of a solution incubated in the absence of the sample and in the presence of the enzyme; B represents the absorption at 320nm of a solution incubated in the absence of both the sample and the enzyme; C represents the absorption at 320nm of a solution incubated in the presence of both the sample and the enzyme; and D represents the absorption at 320nm of a solution incubated in the presence of the sample and in the absence of the enzyme.

Next, the concentration of each sample solution was decreased stepwise and the MMP-1 inhibition (%) was measured. IC$_{50}$ ($\mu$g/mL) (the concentration that results in 50% inhibition) was determined by curve fitting (i.e., the smaller the IC$_{50}$ value, the more potent the elastase inhibitory activity of a given MMP-1 sample). The results are shown in Table 11.

Table 11

|  | IC 50 ($\mu$g/m L) |
|---|---|
| 50% by mass ethanol extract of bayberry | 29.1 |
| 50% by m ass ethanol extract of Japanese pepper | 142.7 |
| 50% by m ass ethanol extract of cowslip | 103.2 |
| 50% by m ass ethanol extract of hairy agrimony | 75.5 |
| 50% by mass ethanol extract of blueberry | 75.0 |

The results of Table 11 indicate that the bayberry extract, the Japanese pepper extract, the cowslip extract, the hairy agrimony extract and the blueberry extract each have an activity to inhibit MMP-1.

(Example 9)

Test for the activity to enhance the production of type-I collagen

The extracts shown in Table 12 below were used as samples and each sample was tested for the activity to enhance the production of type-I collagen in the following manner.

Normal neonatal human skin fibroblasts (NB1RGB) were cultured in Dulbecco's MEM medium supplemented with 10% by mass FBS. Subsequetnly, cells were harvested by trypsin treatment. The harvested cells were diluted with Dulbecco's MEM medium to a cell density of $1.6 \times 10^5$ cells/ml. 100$\mu$L of diluted cells were plated in each well of a 96-well microplate and cells were incubated overnight. After the incubation period, the medium was discarded and 150$\mu$L of a solution of each sample dissolved in Dulbecco's MEM medium supplemented with 0.25% by mass FBS (sample conc. = 100$\mu$g/mL) was added to each well. The microplate was then incubated for 3 days. Subsequently, the amount of type-I collagen in each well was determined by ELISA.

The enhancement rate of type-I collagen production was determined relative to the amount of type-I collagen produced in a sample-free cell culture (= 100%). The enhancement rate of type-I collagen production was determined by the following equation:

$$\% \text{ enhancement of type-I collagen production} = A/B \times 100$$

In the equation above, A represents the amount of type-I collagen produced in a cell culture in the presence of the sample; and B represents the amount of type-I collagen produced in a cell culture in the absence of the sample.

Table 12

|  | % enhancement at sample conc. of 100 $\mu$g/ml |
|---|---|
| 50% by mass ethanol extract of Japanese pepper | 155.9 |
| 50% by mass ethanol extract of cowslip | 173.2 |
| 50% by m ass ethanol extract of lem on verbena | 169.8 |
| 50% by mass ethanol extract of hairy agrimony | 169.8 |
| 50% by m ass ethanol extract of passion fruit | 161.2 |
|  |  |
|  | % enhancement at sample conc. of 25 $\mu$g/ml |
| 50% by m ass ethanol extract of salt cedar | 167.8 |

The results of Table 12 indicate that the Japanese pepper extract, the cowslip extract, the lemon verbena extract, the hairy agrimony extract, the passion fruit extract and the salt cedar extract each have an activity to enhance the production of type-I collagen.

(Example 10)

Test for the activity to enhance the production of type-IV collagen

50% by mass ethanol extract of Japanese pepper was used as a sample and the sample was tested for the activity to enhance the production of type-IV collagen in the following manner.

Normal neonatal human skin fibroblasts (NB1RGB) were cultured in Dulbecco's MEM medium supplemented with 10% by mass FBS. Subsequetnly, cells were harvested by trypsin treatment. The harvested cells were diluted with Dulbecco's MEM medium to a cell density of $1.6 \times 10^5$ cells/ml. 100$\mu$L of diluted cells were plated in each well of a 96-well microplate and cells were incubated overnight. After the incubation period, the medium was discarded and 150$\mu$L of a solution of the sample dissolved in Dulbecco's MEM medium supplemented with 0.25% by mass FBS (sample conc. = 100$\mu$g/mL) was added to each well. The microplate was then incubated for 3 days. Subsequently, the amount of type-IV collagen in each well was determined by ELISA.

The results were used in the following equation 5 to determine the enhancement rate (%) of type-IV collagen.

Table 13 shows the results for the enhancement of type-IV collagen production at a sample concentration of $100\mu g/mL$.

(Equation 5)

$$\% \text{ enhancement of type-IV collagen production} = (A/B) \times 100$$

In the equation 5, A represents the amount of type-IV collagen produced in a cell culture in the presence of the sample; and B represents the amount of type-IV collagen produced in a cell culture in the absence of the sample.

Table 13

|  | % enhancement at sample conc. of $100\mu g/mL$ |
|---|---|
| 50% by mass ethanol extract of Japanese pepper | 144.9 |

The results of Table 13 indicate that the Japanese pepper extract has an activity to enhance the production of type-IV collagen.

(Example 11)

Test for the activity to suppress damage caused by hydrogen peroxide

The extracts shown in Table 14 below were used as samples and each sample was tested for the activity to suppress damage caused by hydrogen peroxide in the following manner.
Normal neonatal human skin fibroblasts (NB1RGB) were cultured in $\alpha$-MEM medium (GIBCO BLR, pH7.2) and cells were subsequently harvested by trypsin treatment. The harvested cells were diluted with $\alpha$-MEM medium to a concentration of $2.5 \times 10^5$ cells/ml. $200\mu L$ of diluted cells were plated in each well of a 48-well plate and cells were incubated overnight. After the incubation period, the medium was discarded and $200\mu L$ of each sample dissolved in $\alpha$-MEM medium supplemented with 1% by mass FBS was added to each well. The plate was then incubated for 24 hours. After the incubation period, the medium was discarded and the plate was rinsed with $400\mu L$ PBS (-). Subsequently, $200\mu L$ of hydrogen peroxide dissolved in Hank's buffer (1mmol/L) or Hank's buffer alone was added to each well and the plate was incubated for 2 hours. Subsequently, the plate was rinsed with $400\mu L$ PBS (-) and $200\mu L$ of neutral red dissolved in $\alpha$-MEM supplemented with 1% by mass FBS to a final concentration of 0.05mg/mL was added to each well. The plate was incubated for 2.5 hours and the neutral red solution was discarded. $200\mu L$ of an ethanol/acetic acid solution (ethanol: acetic acid: water = 50: 1: 49) was then added to each well to extract the dye. The absorption at 540nm was then measured. The results were used in the following equation 6 to determine the suppression rate against damage caused by hydrogen peroxide. The results are shown in Table 14.

(Equation 6)

$$\% \text{ suppression of damage caused by hydrogen peroxide} = \{1 - (C-A)/(C-B)\} \times 100$$

In the equation 6, A represents the absorbance of a cell culture treated with both hydrogen peroxide and the sample; B represents the absorbance of a cell culture treated with hydrogen peroxide but not with the sample; and C represents the absorbance of a cell culture treated with neither hydrogen peroxide nor the sample.

Table 14

|  | % suppression at sample conc. of 100 $\mu g/mL$ |
|---|---|
| 50% by mass ethanol extract of celery | 12.5 |
|  |  |

(continued)

|  | % suppression at sample conc. of 400 $\mu$g/mL |
|---|---|
| 50% by mass ethanol extract of salt cedar | 62.2 |
| 50% by mass ethanol extract of blueberry | 21.3 |

The results of Table 14 indicate that the celery extract, the salt cedar extract and the blueberry extract each have an activity to suppress damage caused by hydrogen peroxide.

(Example 12)

Test for the activity to enhance the production of laminin-5

The extracts shown in Table 15 below were used as samples and each sample was tested for the activity to enhance the production of laminin-5 in the following manner.

Normal human neonatal epidermal keratinocytes (NHEK) were cultured in an 80cm$^2$ flask containing EpiLife-KG2 medium (a culture medium designed for long-term culture of NHEK) at 37°C under 5% $CO_2$. Cells were then harvested by trypsin treatment. The harvested cells were diluted with EpiLife-KG2 to a cell density of $1.0 \times 10^5$ cells/ml. 200$\mu$L of diluted cells were plated in each well of a 24-well plate and the cells were incubated at 37°C under 5% $CO_2$ overnight.

After the incubation period, the medium was discarded and 500$\mu$L of a solution in which each sample was dissolved in EpiLife-KG-2 was added to each well and was incubated at 37°C under 5% $CO_2$ for 48 hours. After the incubation period, 100$\mu$L of the supernatant was transferred to an ELISA plate and was allowed to adsorb onto the plate at 4°C overnight. The solution was then discarded and the plate was washed with a phosphate-buffered solution containing 0.05% by mass Tween-20 (PBS-T). Subsequently, PBS containing 1% by mass bovine serum albumin was added for blocking. The solution was discarded and the plate was washed with a phosphate-buffered solution containing 0.05% by mass Tween-20 (PBS-T). Then, anti-human laminin-5 antibody (mouse IgG, Chemicon) was added for reaction, followed by discarding the solution and washing with a phosphate-buffered solution containing 0.05% by mass Tween-20 (PBS-T). Biotin-labeled anti-mouse IgG antibody (Amersham Bioscience) was then added for reaction. The solution was then discarded and the plate was washed with a phosphate-buffered solution containing 0.05% by mass Tween-20 (PBS-T). Avidin-biotin-peroxidase complex was then added for reaction and the plate was washed again. The color was then developed.

The enhancement rate of laminin-5 production was determined relative to the absorption for a cell culture incubated in the absence of the sample (= 100%). The enhancement rate (%) for each sample was shown in Table 15.

Table 15

|  | % enhancement at sample conc. of 50 $\mu$g/mL |
|---|---|
| 50% by mass ethanol extract of blue flag | 389.7 |
| 50% by mass ethanol extract of passion fruit | 120.6 |
|  |  |
|  | % enhancement at sample conc. of 12.5 $\mu$g/mL |
| 50% by mass ethanol extract of celery | 120.8 |

The results of Table 15 indicate that the blue flag extract, the passion fruit extract and the celery extract each have an activity to enhance the production of laminin-5 by the keratinocytes.

(Example 13)

Test for the activity to enhance the production of transglutaminase-1

The extracts shown in Table 16 below were used as samples and each sample was tested for the activity to enhance the production of transglutaminase-1 in the following manner.

Normal human neonatal epidermal keratinocytes (NHEK) were cultured in EpiLife-KG2 medium (a culture medium designed for long-term culture of NHEK). Cells were then harvested by trypsin treatment. The harvested cells were diluted with EpiLife-KG2 to a concentration of $1.0 \times 10^5$ cells/ml. 100μL of diluted cells were plated in each well of a 96-well plate and cells were incubated for 2 days. After the incubation period, 100μL of each sample dissolved in EpiLife-KG2 was added to each well and the plate was incubated for 24 hours. After the incubation period, the medium was removed and cells were immobilized on the plate. The amount of transglutaminase-1 expressed on the cell surface was determined by ELISA using monoclonal anti-human transglutaminase-1 antibody. The results are shown in Table 16.

The enhancement of transglutaminase-1 production was determined by the following equation:

$$\% \text{ enhancement of transglutaminase-1 production} = A/B \times 100$$

In the equation above, A represents the absorbance at 405nm of a cell culture incubated with the sample; and B represents the absorbance at 405nm of a cell culture incubated in the absence of the sample (Control).

Table 16

|  | % enhancement at sample conc. of 50 μg/mL |
|---|---|
| 50% by mass ehtanol exract of blue flag | 119.5 |
| 50% by mass ehtanol exract of Japanese pepper | 120.5 |
| 50% by mass ehtanol exract of celery | 131.8 |
|  |  |
|  | % enhancement at sample conc. of 12.5 μg/mL |
| 50% by mass ehtanol exract of cowslip | 120.5 |
| 50% by mass ehtanol exract of lemon verbena | 124.9 |
| 50% by mass ehtanol exract of hairy agrimony | 129.5 |
| 50% by mass ehtanol exract of blueberry | 135.1 |

The results of Table 16 indicate that the blue flag extract, the Japanese pepper extract, the celery extract, the cowslip extract, the lemon verbena extract, the hairy agrimony extract and the blueberry extract each have an activity to enhance the production of transglutaminase-1.

(Example 14)

Test for the activity to enhance recovery from damage caused by UVB radiation

The extracts shown in Table 17 below were used as samples and each sample was tested for the activity to enhance recovery from damage caused by UVB radiation in the following manner.

Normal neonatal human skin fibroblasts (NB1RGB) were cultured in α-MEM medium containing 10% by mass FBS and cells were subsequently harvested by trypsin treatment. The harvested cells were diluted with α-MEM to a concentration of $2.0 \times 10^5$ cells/ml. 200μL of diluted cells were plated in each well of a 48-well plate and cells were incubated for 24 hours. Subsequently, the medium was replaced with 100μL PBS(-) and cells were irradiated with UVB radiation at 1.0J/cm$^2$. After radiation, PBS(-) was immediately removed. 400μL of each sample dissolved in D-MEM containing 10% by mass FBS was added to each well and the plate was incubated for 24 hours. The enhancement of recovery from UVB damage was measured by MTT assay. After incubation, the medium was removed and 200μL of MTT dissolved to a final concentration of 0.4mg/mL was added to each well. Following a two-hour incubation period, blue formazan produced in cells was extracted with 200μL 2-propanol. Subsequently, the absorbance was measured at 570nm. The absorbance at 650nm was also measured as the turbidity. The difference between the two values was then taken to give a measurement of the blue formazan production. Cells similarly plated but not irradiated with UVB radiation and cells similarly plated, irradiated with UVB radiation, but in the absence of the sample were also analyzed in the same manner and were designated as a non-radiation group and a radiation group, respectively.

The activity to enhance recovery from UVB damage was determined by the following equation. The results are shown in Table 17.

$$\text{\% enhancement of recovery from UVB damage} = \{(Nt \cdot C) \cdot (Nt \cdot Sa)\}/(Nt \cdot C) \times 100$$

In the equation above, Nt represents the absorbance of a cell culture incubated without UVB radiation; C represents the absorbance of a cell culture incubated with UVB radiation but in the absence of the sample; and Sa represents absorbance of a cell culture incubated with UVB radiation in the presence of the sample.

Table 17

|  | % recovery at sample conc. of 100 $\mu$g/mL |
|---|---|
| 50% by mass ethanol extract of Japanese pepper | 38.6 |
| 50% by mass ethanol extract of hairy agrimony | 25.1 |
| 50% by mass ethanol extract of blueberry | 15.0 |
| 50% by mass ethanol extract of celery | 15.8 |

The results of Table 17 indicate that the Japanese pepper extract, the hairy agrimony extract, the blueberry extract and the celery extract each have an activity to enhance recovery from damage caused by UVB radiation.

(Example 15)

Test for the activity to enhance the growth of epidermal keratinocytes

50% by mass ethanol extract of cowslip was used as a sample and was tested for its activity to enhance the growth of epidermal keratinocytes in the following manner.

Normal human neonatal epidermal keratinocytes (NHEK) were cultured in EpiLife-KG2 medium (a culture medium designed for long-term culture of NHEK). Cells were then harvested by trypsin treatment. The harvested cells were diluted with EpiLife-KG2 to a concentration of $2.0 \times 10^4$ cells/ml. 100$\mu$L of diluted cells were plated in each well of a collagen-coated 96-well plate and cells were incubated overnight. After the incubation period, 100$\mu$L of the sample dissolved in EpiLife-KG2 was added to each well and the plate was incubated for 3 days.

The activity to enhance the growth of epidermal keratinocytes was measured by MTT assay. After incubation, the medium was removed and 100$\mu$L of MTT (3-(4,5-Dimethyl-2-thiazolyl)-2,5-diphenyl-2H-tetrazolium Bromide) dissolved in PBS(-) to a final concentration of 0.4mg/mL was added to each well. Following a two-hour incubation period, blue formazan produced in cells was extracted with 100$\mu$L 2-propanol. Subsequently, the absorbance was measured at 570nm. The absorbance at 650nm was also measured as the turbidity. The difference between the two values was then taken to give a measurement of the blue formazan production. A blank test was also conducted in the same manner for correction.

The results were used in the following equation 7 to determine the enhancement rate of the growth of epidermal keratinocytes. The enhancement rate of the growth of epidermal keratinocytes at a sample concentration of 3.13$\mu$g/mL is shown in Table 18.

(Equation 7)

$$\text{\% enhancement of the growth of epidermal keratinocytes} = (St/Ct) \times 100$$

In the equation 7, St represents the absorbance of a cell culture incubated in the presence of the sample; and Ct represents the absorbance of a cell culture incubated in the absence of the sample.

Table 18

|  | % enhancemnt at sample conc. of 3.13$\mu$g/mL |
|---|---|
| 50% by mass ethanol extract of cowslip | 114.0 |

The results of Table 18 indicate that the cowslip extract has an activity to enhance the growth of epidermal keratinocytes.

(Example 16)

Test for the activity to enhance the growth of skin fibroblasts

50% by mass ethanol extract of Japanese pepper was used as a sample and was tested for its activity to enhance the growth of skin fibroblasts in the following manner.

Normal neonatal human skin fibroblasts (NB1RGB) were cultured in $\alpha$-MEM medium containing 10% by mass FBS and cells were subsequently harvested by trypsin treatment. The harvested cells were diluted with $\alpha$-MEM containing 5% by mass FBS to a concentration of $7.0 \times 10^4$ cells/ml. 100$\mu$L of diluted cells were plated in each well of a 96-well plate and cells were incubated overnight.

After the incubation period, 100$\mu$L of the sample dissolved in $\alpha$-MEM containing 5% by mass FBS (conc. = 400$\mu$g/mL) was added to each well and the plate was incubated for 3 days.

The activity to enhance the growth of skin fibroblasts was measured by MTT assay. Specifically, 100$\mu$L medium was removed from each well and 20$\mu$L of MTT (3-(4,5-Dimethyl-2-thiazolyl)-2,5-diphenyl-2H-tetrazolium Bromide) dissolved in PBS(-) to a final concentration of 5mg/mL was added to each well. Following a 4.5-hour incubation period, 100$\mu$L of a 0.01mol/L hydrochloric acid solution of SDS (10% by mass) was added to each well and the plate was incubated overnight. Subsequently, the absorbance was measured at 570nm. The absorbance at 650nm was also measured as the turbidity. The difference between the two values was then taken to give a measurement of the blue formazan production. A blank test was also conducted in the same manner for correction.

The results were used in the following equation 8 to determine the enhancement rate of the growth of skin fibroblasts at a sample concentration of 200 $\mu$g/mL. The results are shown in Table 19.

$$\text{(Equation 8)}$$

$$\% \text{ enhancement of the growth of skin fibroblasts} = (St\text{-}Sb)/(Ct\text{-}Cb) \times 100$$

In the equation 8, St represents the absorbance of a cell culture incubated in the presence of the sample; Sb represents the absorbance of a blank incubated in the presence of the sample; Ct is the absorbance of a cell culture incubated in the absence of the sample; and Cb is the absorbance of a blank incubated in the absence of the sample.

Table 19

|  | % enhancement at sample conc. of 200 $\mu$g/mL |
|---|---|
| 50% by mass ethanol extract of Japanese pepper | 130.6 |

The results of Table 19 indicate that the Japanese pepper extract has an activity to enhance the growth of skin fibroblasts.

(Example 17)

Test for the activity to enhance the production of involucrin

50% by mass ethanol extract of cowslip was used as a sample and was tested for its activity to enhance the production of involucrin in the following manner.

Normal human neonatal epidermal keratinocytes (NHEK) were cultured in an 80cm$^2$ flask containing EpiLife-KG2 medium (a culture medium designed for long-term culture of NHEK) at 37°C under 5% $CO_2$. Cells were then harvested by trypsin treatment. The harvested cells were diluted with EpiLife-KG2 to a cell density of $1.0 \times 10^5$ cells/ml. 200$\mu$L of diluted cells were plated in each well of a 48-well plate and cells were incubated at 37°C under 5% $CO_2$ overnight.

After the incubation period, the medium was removed and 200$\mu$L of the sample dissolved in EpiLife-KG2 was added to each well and the plate was incubated at 37°C under 5% $CO_2$ for 48 hours. After the incubation period, the medium was removed and cells were immobilized on the plate. The amount of involucrin expressed on the cell surface was measured by ELISA using monoclonal anti-human involucrin antibody. The results were used in the following equation 9 to determine the enhancement rate (%) of involucrin production. The results are shown in Table 20.

(Equation 9)

$$\% \text{ enhancement of involucrin production} = A/B \times 100$$

In the equation 9, A represents the absorbance at 405nm of a cell culture incubated in the presence of the sample; and B represents the absorbance at 405nm of a cell culture incubated in the absence of the sample.

Table 20

|  | % enhancement at sample conc. of 12.5 $\mu$g/mL |
|---|---|
| 50% by mass ethanol extract of cowslip | 149.1 |

The results of Table 20 indicate that the cowslip extract has a high activity to enhance the production of involucrin.

(Example 18)

Test for the activity to enhance the production of adenosine triphosphate (ATP)

The extracts shown in Table 21 below were used as samples and each sample was tested for the activity to enhance ATP production in the following manner.

Normal human neonatal epidermal keratinocytes (NHEK) were cultured in EpiLife-KG2 medium (a culture medium designed for long-term culture of NHEK). Cells were then harvested by trypsin treatment. The harvested cells were diluted with EpiLife-KG2 to a concentration of $2.0 \times 10^5$ cells/ml. 100$\mu$L of diluted cells were plated in each well of a collagen-coated 96-well plate and cells were incubated overnight. After the incubation period, the medium was removed and 100$\mu$L of each sample dissolved in EpiLife-KG2 was added to each well and the plate was incubated for 2 hours. The activity to enhance ATP production was measured by determining the amount of ATP in cells by the firefly luciferase luminescence assay. Specifically, after the incubation period, 100$\mu$L of cell ATP assay reagents (Toyo B-net) was added to each well. The level of the resulting chemiluminescence was measured after the reaction. The results are shown in Table 21.

The enhancement rate of ATP production was determined by the following equation.

$$\% \text{ enhancement of ATP production} = A/B \times 100$$

In the equation above, A represents the level of chemiluminescence of a cell culture incubated in the presence of the sample; and B represents the level of chemiluminescence of a cell culture incubated in the absence of the sample.

Table 21

|  | % enhancement at sample conc. of 20 $\mu$g/mL |
|---|---|
| 50% by mass ethanol extract of bayberry | 110.5 |
|  |  |
|  | % enhancement at sample conc. of 5 $\mu$g/mL |
| 50% by mass ethanol extract of lemon verbena | 109.5 |

The results of Table 21 indicate that the bayberry extract and the lemon verbena extract both have an activity to enhance ATP production.

(Example 19)

Test for the activity to enhance the production of profilaggrin and filaggrin

The extracts shown in Table 22 below were used as samples and each sample was tested for the activity to enhance the production of profilaggrin and filaggrin in the following manner.

Normal human neonatal epidermal keratinocytes (NHEK) were cultured in EpiLife-KG2 medium (a culture medium designed for long-term culture of NHEK). Cells were then harvested by trypsin treatment. The harvested cells were diluted with the same medium to a cell density of $1.5 \times 10^5$ cells/ml. 2mL of the diluted cell solution was plated in each well of a 6-well collagen-coated plate and the plate was incubated at 37°C under 5% $CO_2$-95% air for 3 days. After the incubation period, the medium was replaced with 2mL of EpiLife-KG2 containing the sample dissolved in 0.5% by mass DMSO (Sample concentrations are shown in Table 22). The plate was incubated at 37°C under 5% $CO_2$-95% air for 5 days. After the incubation period, total protein was prepared by a commonly used technique.

(Western blotting)

Each sample was loaded on SDS-PAGE at 10μg/lane and was separated. The separated sample was transferred to a PVDF membrane. After blocking with PBS(-) containing 5% skim milk, an anti-human filaggrin monoclonal antibody (Habor Bioproducts), a biotin-labeled anti-mouse Ig (whole Ab, Amersham Biosciences), and a streptavidin-peroxidase complex (Calbiochem), each diluted 1000-fold with PBS(-) containing 0.1% Tween and 0.3%.skim milk, were sequentially reacted. Profilaggrin and filaggrin were detected by luminescence using ECL Western blotting detection reagents and analysis system (Amersham Biosciences). The detected bands were quantitatively analyzed by KODAK 1D Image Analysis Software EDAS290 Version3.5.

The band intensities for profilaggrin and filaggrin present in the respective 10μg protein preparations prepared from the respective cell cultures incubated with or without each sample were added together to give net intensities that served as a measure of the ability of the sample to enhance profilaggrin production. The enhancement rate (%) of profilaggrin production was determined by the following equation. The results are shown in Table 22.

$$\% \text{ enhancement of ATP production} = A/B \times 100$$

In the equation above, A represents the net intensity (i.e., the combined value for profilaggrin and filaggrin) for a cell culture incubated in the presence of the sample; and B represents the net intensity for a cell culture incubated in the absence of the sample (Control).

Table 22

|  | % enhancement at sample conc. of 12.5μg/ml |
|---|---|
| 50% by mass ethanol extract of blue flag | 129.8 |
| 50% by mass ethanol extract of passion fruit | 121.6 |

The results of Table 22 indicate that the blue flag extract and the passion fruit extract both have an activity to enhance the production of filaggrin.

(Example 20)

Test for the activity to enhance the expression of hyaluronic acid synthase 3 (HAS3) mRNA

The extracts shown in Table 23 below were used as samples and each sample was tested for the activity to enhance the expression of hyaluronic acid synthase 3 (HAS3) mRNA in the following manner.

Normal human neonatal epidermal keratinocytes (NHEK) were pre-cultured in an 80cm² flask containing EpiLife-KG2 medium (a culture medium designed for long-term culture of NHEK) at 37°C under 5% $CO_2$-95% air. Cells were then harvested by trypsin treatment.

2mL each of the harvested cells ($40 \times 10^4$ cells) were then plated onto EpiLife-KG2 in a 35mm petri dish (FALCON) and were incubated at 37°C under 5% $CO_2$-95% air overnight. After 24 hours, the medium was discarded and 2mL of each of the samples dissolved in EpiLife-KG2 (Concentrations are shown in Table 23) was added to each petri dish and cells were further incubated at 37°C under 5% $CO_2$-95% air for 24 hours. After the incubation period, the medium was discarded and total RNA was extracted using ISOGEN (Nippon Gene, Cat. No. 311-02501). The amount of RNA for each culture was measured by a spectrophotometer and total RNA was prepared to a concentration of 200ng/μL.

Using the total RNA as a template, the expression levels of hyaluronic acid synthase 3 (HAS3) mRNA and GAPDH mRNA to serve as the internal standard were determined. The detection was carried out by performing real-time 2-step RT-PCR using Takara SYBR Prime Script RT-PCR Kit (Perfect Real Time, code No. RR063A) on a Smart Cycler real-time PCR system (Cepheid). The expression levels of HAS3 were determined as follows: total RNA was obtained from cells incubated in the absence of the sample and cells incubated in the presence of the sample. The expression levels of the respective total RNA were corrected relative to GAPDH expression. The expression levels for cells incubated in the presence of the sample were then corrected relative to the expression levels for cells incubated in the absence of the sample (= 100). The results were used in the following equation to determine the enhancement rate (%) of the HAS3 mRNA expression for each sample. The results are shown in Table 23.

$$\text{\% enhancement of HAS3 mRNA expression} = A/B \times 100$$

In the equation above, A represents the corrected expression level for cells incubated in the presence of the sample; and B represents the corrected expression level for cells incubated in the absence of the sample.

Table 23

|  | % enhancement at sample conc. of 12.5 $\mu$g/ml |
|---|---|
| 50% by mass ethanol extract of Japanese pepper | 212.6 |
| 50% by mass ethanol extract of celery | 127.4 |
|  |  |
|  | % enhancement at sample conc. of 50 $\mu$g/ml |
| 50% by mass ethanol extract of blue flag | 177.6 |
| 50% by mass ethanol extract of lemon verbena | 124.9 |

The results of Table 23 indicate that the Japanese pepper extract, the blue flag extract, the celery extract and the lemon verbena extract each have an activity to enhance the expression of hyaluronic acid synthase 3 (HAS3) mRNA.

(Example 21)

Test for the activity to enhance the expression of aquaporin-3 (AQP3) mRNA

The extracts shown in Table 24 below were used as samples and each sample was tested for the activity to enhance the expression of aquaporin-3 (AQP3) mRNA in the following manner.
Normal human neonatal epidermal keratinocytes (NHEK) were pre-cultured in an 80cm$^2$ flask containing EpiLife-KG2 medium (a culture medium designed for long-term culture of NHEK) at 37°C under 5% $CO_2$ Cells were then harvested by trypsin treatment.
The harvested cells were then plated onto EpiLife-KG2 in a 35mm petri dish (FALCON) at $40 \times 10^4$ cells/2mL/dish and were incubated at 37°C under 5% $CO_2$ overnight. After 24 hours, the medium was discarded and 2mL of each of the samples dissolved in EpiLife-KG2 to a desired concentration was added to each petri dish and cells were further incubated at 37°C under 5% $CO_2$ for 24 hours. After the incubation period, the medium was discarded and total RNA was extracted using ISOGEN (Nippon Gene, Cat. No. 311-02501). The amount of RNA for each culture was measured by a spectrophotometer and total RNA was prepared to a concentration of 200 ng/$\mu$L.
Using the total RNA as a template, the expression levels of AQP3 mRNA and GAPDH mRNA to serve as the internal standard were determined. The detection was carried out by performing real-time 2-step RT-PCR using Takara SYBR® PrimeScript® RT-PCR Kit (Perfect Real Time)(code No. RR063A) on a Smart Cycler® real-time PCR system (Cepheid). The expression levels of AQP3 were determined as follows: total RNA was obtained from cells incubated in the absence of the sample and cells incubated in the presence of the sample. The expression levels of the respective total RNA were corrected relative to GAPDH expression. The expression levels for cells incubated in the presence of the sample were then corrected relative to the expression levels for cells incubated in the absence of the sample (= 100). The results are shown in Table 24.
The activity to enhance AQP3 mRNA expression was determined by the following equation.

$$\% \text{ enhancement of AQP3 mRNA expression} = A/B \times 100$$

In the equation above, A represents the corrected expression level for cells incubated in the presence of the sample; and B represents the corrected expression level for cells incubated in the absence of the sample.

Table 24

|  | % enhancement at sample conc. of 50 $\mu$g/mL |
|---|---|
| 50% by mass ethanol extract of blue flag | 278.9 |
| 50% by mass ethanol extract of cowslip | 149.2 |
| 50% by mass ethanol extract of celery | 135.1 |

The results of Table 24 indicate that the blue flag extract, the cowslip extract and the celery extract each have an activity to enhance the expression of aquaporin-3 (AQP3) mRNA.

(Formulation Example 1)

Emulsion

An emulsion having the following composition was produced using a common process.

- jojoba oil        4.0 g
- placenta extract        0.1 g
- olive oil        2.0 g
- squalane        2.0 g
- cetanol        2.0 g
- glyceryl monostearate        2.0 g
- polyoxyethylene cetyl ether (20E.O)        2.5 g
- polyoxyethylene sorbitan oleate (20E.O)        2.0 g
- 1,3-butylene glycol        3.0 g
- hinokitiol        0.15 g
- perfume        0.05 g
- 50% by mass ethanol extract of bayberry        0.01 g

(Production Example 2)

- purified water        Balance (Total = 100 g)

(Formulation Example 2)

Emulsion

An emulsion having the following composition was produced using a common process.

- jojoba oil        4.0 g
- placenta extract        0.1 g
- olive oil        2.0 g
- squalane        2.0 g
- cetanol        2.0 g
- glyceryl monostearate        2.0 g
- polyoxyethylene cetyl ether (20E.O)        2.5 g
- polyoxyethylene sorbitan oleate (20E.O)        2.0 g

- 1,3-butylene glycol        3.0 g
- hinokitiol        0.15 g
- perfume        0.05 g
- 50% by mass ethanol extract of blue flag        0.01 g

(Production Example 2)

- purified water        Balance (Total = 100 g)

(Formulation Example 3)

Emulsion

An emulsion having the following composition was produced using a common process.

- jojoba oil        4.0 g
- placenta extract        0.1 g
- olive oil        2.0 g
- squalane        2.0 g
- cetanol        2.0 g
- glyceryl monostearate        2.0 g
- polyoxyethylene cetyl ether (20E.O)        2.5 g
- polyoxyethylene sorbitan oleate (20E.O)        2.0 g
- 1,3-butylene glycol        3.0 g
- hinokitiol        0.15 g
- perfume        0.05 g
- 50% by mass ethanol extract of Japanese        0.01 g pepper (Production Example 2)
- purified water        Balance (Total = 100 g)

(Formulation Example 4)

Emulsion

An emulsion having the following composition was produced using a common process.

- jojoba oil        4.0 g
- placenta extract        0.1 g
- olive oil        2.0 g
- squalane        2.0 g
- cetanol        2.0 g
- glyceryl monostearate        2.0 g
- polyoxyethylene cetyl ether (20E.O)        2.5 g
- polyoxyethylene sorbitan oleate (20E.O)        2.0 g
- 1,3-butylene glycol        3.0 g
- hinokitiol        0.15 g
- perfume        0.05 g
- 50% by mass ethanol extract of cowslip        0.01 g

(Production Example 2)

- purified water        Balance (Total = 100 g)

(Formulation Example 5)

Emulsion

An emulsion having the following composition was produced using a common process.

- jojoba oil          4.0 g
- placenta extract          0.1 g
- olive oil          2.0 g
- squalane          2.0 g
- cetanol          2.0 g
- glyceryl monostearate          2.0 g
- polyoxyethylene cetyl ether (20E.O)          2.5 g
- polyoxyethylene sorbitan oleate (20E.O)          2.0 g
- 1,3-butylene glycol          3.0 g
- hinokitiol          0.15 g
- perfume          0.05 g
- 50% by mass ethanol extract of lemon          0.01 g verbena (Production Example 2)
- purified water          Balance (Total = 100 g)

(Formulation Example 6)

Emulsion

An emulsion having the following composition was produced using a common process.

- jojoba oil          4.0 g
- placenta extract          0.1 g
- olive oil          2.0 g
- squalane          2.0 g
- cetanol          2.0 g
- glyceryl monostearate          2.0 g
- polyoxyethylene cetyl ether (20E.O)          2.5 g
- polyoxyethylene sorbitan oleate (20E.O)          2.0 g
- 1,3-butylene glycol          3.0 g
- hinokitiol          0.15 g
- perfume          0.05 g
- 50% by mass ethanol extract of hairy          0.01 g agrimony (Production Example 2)
- purified water          Balance (Total = 100 g)

(Formulation Example 7)

Emulsion

An emulsion having the following composition was produced using a common process.

- jojoba oil          4.0 g
- placenta extract          0.1 g
- olive oil          2.0 g
- squalane          2.0 g
- cetanol          2.0 g
- glyceryl monostearate          2.0 g
- polyoxyethylene cetyl ether (20E.O)          2.5 g
- polyoxyethylene sorbitan oleate (20E.O)          2.0 g
- 1,3-butylene glycol          3.0 g
- hinokitiol          0.15 g
- perfume          0.05 g
- 50% by mass ethanol extract of blueberry          0.01 g

(Production Example 2)

• purified water        Balance (Total = 100 g)

(Formulation Example 8)

Emulsion

An emulsion having the following composition was produced using a common process.

- • jojoba oil        4.0 g
- • placenta extract        0.1 g
- • olive oil        2.0 g
- • squalane        2.0 g
- • cetanol        2.0 g
- • glyceryl monostearate        2.0 g
- • polyoxyethylene cetyl ether (20E.O)        2.5 g
- • polyoxyethylene sorbitan oleate (20E.O)        2.0 g
- • 1,3-butylene glycol        3.0 g
- • hinokitiol        0.15 g
- • perfume        0.05 g
- • 50% by mass ethanol extract of celery        0.01 g

(Production Example 2)

• purified water        Balance (Total = 100 g)

(Formulation Example 9)

Emulsion

An emulsion having the following composition was produced using a common process.

- • jojoba oil        4.0 g
- • placenta extract        0.1 g
- • olive oil        2.0 g
- • squalane        2.0 g
- • cetanol        2.0 g
- • glyceryl monostearate        2.0 g
- • polyoxyethylene cetyl ether (20E.O)        2.5 g
- • polyoxyethylene sorbitan oleate (20E.O)        2.0 g
- • 1,3-butylene glycol        3.0 g
- • hinokitiol        0.15 g
- • perfume        0.05 g
- • 50% by mass ethanol extract of salt cedar        0.01 g

(Production Example 2)

• purified water        Balance (Total = 100 g)

(Formulation Example 10)

Emulsion

An emulsion having the following composition was produced using a common process.

- jojoba oil          4.0 g
- placenta extract          0.1 g
- olive oil          2.0 g
- squalane          2.0 g
- cetanol          2.0 g
- glyceryl monostearate          2.0 g
- polyoxyethylene cetyl ether (20E.O)          2.5 g
- polyoxyethylene sorbitan oleate (20E.O)          2.0 g
- 1,3-butylene glycol          3.0 g
- hinokitiol          0.15 g
- perfume          0.05 g
- 50% by mass ethanol extract of passion fruit          0.01 g

(Production Example 2)

- purified water          Balance (Total = 100 g)

(Formulation Example 11)

Cream

A cream having the following composition was produced using a common process.

- liquid paraffin          5.0 g
- white beeswax          4.0 g
- cetanol          3.0 g
- squalane          10.0 g
- lanolin          2.0 g
- stearic acid          1.0 g
- polyoxyethylene sorbitan oleate (20E.O)          1.5 g
- glyceryl monostearate          3.0 g
- 1,3-butylene glycol          6.0 g
- methyl p-oxybenzoate          1.5 g
- perfume          0.1 g
- 80% by mass ethanol extract of blue flag          0.1 g

(Production Example 3)

- purified water          balance (Total = 100 g)

(Formulation Example 12)

Pack

A pack having the following composition was produced using a common process.

- polyvinyl alcohol          15 g
- polyethylene glycol          3 g

- propylene glycol        7 g
- ethanol        10 g
- ethyl p-oxybenzoate        0.05 g
- perfume        0.05 g
- water extract of Japanese pepper        0.05 g

(Production Example 1)

- purified water        balance (total = 100 g)

Industrial Applicability

The skin-whitening agent or anti-aging agent of the present invention has at least one of high tyrosinase inhibitory activity, activity to suppress melanin production, activity to suppress the expression of SCF mRNA, activity to suppress the expression of endothelin-1 mRNA, activity to suppress the expression of bFGF mRNA, activity to suppress the expression of POMC mRNA, elastase inhibitory activity, MMP-1 inhibitory activity, activity to enhance ATP production, activity to enhance the production of laminin-5, activity to enhance the production of filaggrin, activity to enhance the production of transglutaminase-1, activity to enhance the expression of mRNA of hyaluronic acid synthase-3 (HAS3), activity to enhance the expression of mRNA of aquaporin-3 (AQP3), activity to enhance the production of type-I collagen, activity to enhance the production of type-IV collagen, activity to enhance the growth of skin fibroblasts, activity to enhance recovery from damage caused by UVB radiation, activity to enhance the growth of epidermal keratinocytes, activity to enhance the production of involucrin and activity to suppress damage caused by hydrogen peroxide. Accordingly, the skin-whitening agent or anti-aging agent of the present invention can be widely used in skin-care cosmetic agents such as ointments, creams, emulsions, lotions, packs, jelly, lip creams, lipsticks, bath agents and astringents.

**Claims**

1. A skin-whitening agent, comprising:

   at least one from the group consisting of bayberry extract, blue flag extract, Japanese pepper extract, cowslip extract, lemon verbena extract, hairy agrimony extract, blueberry extract, celery extract, salt cedar extract and passion fruit extract.

2. The skin-whitening agent according to claim 1, wherein the skin-whitening agent has at least one activity selected from the group consisting of tyrosinase inhibitory activity, activity to suppress melanin production, activity to suppress the expression of SCF mRNA, activity to suppress the expression of endothelin-1 mRNA, activity to suppress the expression of bFGF mRNA and activity to suppress the expression of POMC mRNA.

3. An anti-aging agent, comprising:

   at least one selected from the group consisting of bayberry extract, blue flag extract, Japanese pepper extract, cowslip extract, lemon verbena extract, hairy agrimony extract, blueberry extract, celery extract, salt cedar extract and passion fruit extract.

4. The anti-aging agent according to claim 3, wherein the anti-aging agent has at least one activity selected from the group consisting of elastase inhibitory activity, MMP-1 inhibitory activity, activity to enhance the production of type-I collagen, activity to enhance the production of type-IV collagen, activity to suppress damage caused by hydrogen peroxide, activity to enhance the production of laminin-5, activity to enhance the production of transglutaminase-1, activity to enhance recovery from damage caused by UVB radiation, activity to enhance the growth of epidermal keratinocytes, activity to enhance the growth of skin fibroblasts, activity to enhance the production of involucrin, activity to enhance ATP production, activity to enhance the production of filaggrin, activity to enhance the expression of mRNA of hyaluronic acid synthase-3 (HAS3) and activity to enhance the expression of mRNA of aquaporin-3 (AQP3).

5. A skin-care cosmetic agent, comprising:

at least one of the skin-whitening agent as defined in any of claim 1 or 2, and the anti-aging agent as defined in any of claim 3 or 4.

INTERNATIONAL SEARCH REPORT

| International application No. |
| --- |
| PCT/JP2009/065848 |

A. CLASSIFICATION OF SUBJECT MATTER
*A61K8/97*(2006.01)i, *A61Q19/02*(2006.01)i, *A61Q19/08*(2006.01)i

According to International Patent Classification (IPC) or to both national classification and IPC

B. FIELDS SEARCHED

Minimum documentation searched (classification system followed by classification symbols)
A61K8/97, A61Q19/02, A61Q19/08

Documentation searched other than minimum documentation to the extent that such documents are included in the fields searched
Jitsuyo Shinan Koho         1922-1996   Jitsuyo Shinan Toroku Koho   1996-2009
Kokai Jitsuyo Shinan Koho   1971-2009   Toroku Jitsuyo Shinan Koho   1994-2009

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| P,X<br>P,A | JP 2009-102298 A  (Morinaga & Co., Ltd.),<br>14 May 2009 (14.05.2009),<br>claim 1; paragraphs [0003], [0004]<br>(Family: none) | 1,2,5<br>3,4 |
| P,X<br>P,A | JP 2009-40753 A  (Advangen, Inc.),<br>26 February 2009 (26.02.2009),<br>claims 1 to 4; paragraph [0001]; table 3;<br>fig. 6<br>(Family: none) | 3-5<br>1,2 |
| X | Junji TANAKA, Mitunori KIKUCHI, SHAN Shao-Jie,<br>Hiroshi SHIMODA, SU Muh-Hwan, "Cistanche<br>Tubulosa Extract no Aratana Kenko Kino 6 Biyo<br>Oyobi Ikumo Sayo", Food Style 21, 01 June 2008<br>(01.06.2008), vol.12 no.6, Pages.29 to 32 | 1-5 |

☒  Further documents are listed in the continuation of Box C.        ☐  See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>29 October, 2009 (29.10.09) | Date of mailing of the international search report<br>10 November, 2009 (10.11.09) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
|---|
| PCT/JP2009/065848 |

C (Continuation).    DOCUMENTS CONSIDERED TO BE RELEVANT

| Category* | Citation of document, with indication, where appropriate, of the relevant passages | Relevant to claim No. |
|---|---|---|
| X<br>A | JP 2007-261996 A  (Naris Cosmetics Co., Ltd.),<br>11 October 2007 (11.10.2007),<br>claims 1, 2; paragraph [0001]; tables 1, 2<br>(Family: none) | 3-5<br>1,2 |
| X | JP 2006-75084 A  (Toyo Shinyaku Co., Ltd.),<br>23 March 2006 (23.03.2006),<br>claims 1, 3, 5<br>(Family: none) | 1-5 |
| X | JP 2006-8566 A  (Ichimaru Pharcos Co., Ltd.),<br>12 January 2006 (12.01.2006),<br>claims 1, 2; table 3; fig. 10<br>(Family: none) | 1-5 |
| X<br>A | JP 2005-343884 A  (Coletica),<br>15 December 2005 (15.12.2005),<br>claims 5, 6; example 24<br>& US 2005/0271751 A1     & GB 2414669 A<br>& DE 102004045187 A     & FR 2871061 A<br>& KR 10-2005-0115817 A | 3-5<br>1,2 |
| X<br>A | JP 2005-306850 A  (Kose Corp.),<br>04 November 2005 (04.11.2005),<br>claims 1 to 3, 6<br>(Family: none) | 3-5<br>1,2 |
| X<br>A | JP 2005-132823 A  (L'Oreal),<br>26 May 2005 (26.05.2005),<br>claims 1, 14, 15<br>& US 2005/0058611 A1     & EP 1508328 A1 | 3-5<br>1,2 |
| X | JP 2005-60334 A  (Okinawa-Ken),<br>10 March 2005 (10.03.2005),<br>claims 1, 7; paragraphs [0017], [0057]<br>(Family: none) | 1-5 |
| X<br>A | WO 2003/84302 A1  (Shiseido Co., Ltd.),<br>16 October 2003 (16.10.2003),<br>claims 1 to 3; fig. 1, 2<br>& US 2005/0220810 A1     & EP 1559429 A2<br>& CN 1688326 A | 3-5<br>1,2 |
| X<br>A | JP 2001-316221 A  (Naris Cosmetics Co., Ltd.),<br>13 November 2001 (13.11.2001),<br>claims 1, 4<br>(Family: none) | 3-5<br>1,2 |
| X | JP 11-269081 A  (Pola Chemical Industries Inc.),<br>05 October 1999 (05.10.1999),<br>claim 1; paragraph [0001]<br>(Family: none) | 1-5 |

Form PCT/ISA/210 (continuation of second sheet) (April 2007)

# EP 2 335 685 A1

| International application No. |
|---|
| PCT/JP2009/065848 |

**C (Continuation).** DOCUMENTS CONSIDERED TO BE RELEVANT

| Category* | Citation of document, with indication, where appropriate, of the relevant passages | Relevant to claim No. |
|---|---|---|
| X | JP 9-77636 A (Mikimoto Pharmaceutical Co., Ltd.), 25 March 1997 (25.03.1997), claim 1; preparation example 13 (Family: none) | 1-5 |
| X | JP 5-97648 A (Tsuneo NANBA), 20 April 1993 (20.04.1993), claim 1; paragraph [0005] (Family: none) | 1-5 |
| X A | JP 2005-350483 A (Sunstar Inc.), 22 December 2005 (22.12.2005), claims 1, 2; example 3 & US 6814958 B1 & EP 1072254 A1 & WO 1999/055298 A1 | 3-5 1,2 |
| A | JP 2003-192605 A (Fancl Corp.), 09 July 2003 (09.07.2003), claim 1 (Family: none) | 1-5 |
| A | JP 2005-350391 A (Noevir Co., Ltd.), 22 December 2005 (22.12.2005), claim 1; paragraphs [0014], [0015] (Family: none) | 1-5 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000344672 A
- JP 2002370962 A

- JP 2004083488 A

**Non-patent literature cited in the description**

- *Experimental Dermatology,* 2003, vol. 12, 591-601

- *Fragrance Journal,* 2000, vol. 28 (9), 65-71